Europäisches Patentamt

European Patent Office

Office européen des brevets

(19)

(11) Numéro de publication : **0 354 848 B1**

(12)

# FASCICULE DE BREVET EUROPEEN

(45) Date de publication du fascicule du brevet :
**11.11.92 Bulletin 92/46**

(51) Int. Cl.⁵ : **A61K 31/205, // (A61K31/205, 31:195)**

(21) Numéro de dépôt : **89402257.3**

(22) Date de dépôt : **10.08.89**

(54) **Compositions pharmaceutiques et/ou diététiques contenant de la L-carnitine et de la L-lysine.**

Jointe à la demande no. 89909580.6/0428608 (numéro de dépôt/numéro de publication de la demande européenne) par décision du 04.09.91.

(30) Priorité : **12.08.88 FR 8810886**

(43) Date de publication de la demande :
**14.02.90 Bulletin 90/07**

(45) Mention de la délivrance du brevet :
**11.11.92 Bulletin 92/46**

(84) Etats contractants désignés :
**AT BE CH DE ES FR GB GR IT LI LU NL SE**

(56) Documents cités :
**FR-M- 4 465**
**US-A- 3 830 931**

(56) Documents cités :
**US-A- 3 968 241**
**UNLISTED DRUGS, vol. 23, no. 3, mars 1971, page 41n, Chatham, New Jersey, US; "Regeneron"**
**UNLISTED DRUGS, vol. 23, no. 1, janvier 1971, page 9d, Chatham, New Jersey, US; "Osvical-lisina"**

(73) Titulaire : **Société anonyme dite: LABORATOIRE ROGER BELLON 159, avenue A. Peretti F-92201 Neuilly-sur-Seine (FR)**

(72) Inventeur : **Laborit, Henri 93, rue de la Santé F-75013 Paris (FR)**

(74) Mandataire : **Portal, Gérard et al Cabinet Beau de Loménie 55, rue d'Amsterdam F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 354 848 B1

## Description

La présente invention concerne généralement de nouvelles compositions pharmaceutiques et/ou diététiques contenant de la L-carnitine et de la L-lysine en quantités produisant un effet de potentialisation.

On sait que la carnitine et ses dérivés ont déjà été utilisés pour des applications thérapeutiques nombreuses et variées.

Par exemple, l'utilisation de la D,L-carnitine est bien connue dans les troubles de la nutrition et dans les affections musculaires de type dystrophique. L'utilisation de la D,L-carnitine pour le traitement de l'insuffisance cardiaque est décrite dans les brevets US-A-3 830 931 et 3 968 241.

De même, le document FR-A-2 482 588 décrit l'utilisation de la L-carnitine dans le traitement de patients atteints de diabète sucré juvénile.

On sait par ailleurs que la L-carnitine intervient comme co-facteur et comme agent de protection des processus énergétiques cellulaires, notamment dans les tissus riches en mitochondries, tels que les muscles et le myocarde.

Plus généralement, les propriétés physiologiques et pharmacologiques de la L-carnitine ont fait l'objet des publications récentes suivantes :

- BAHL J. and BRESSLER R. (1987) : The pharmacology of carnitine. Ann. Rev. Pharmacol. Toxicol, <u>27</u> : 257-277 ;
- BORUM P.R. (1983) : Carnitine. Ann. Rev. Nutr. 3 : 233-259
- BREMER J. (1983) : Carnitine. Metabolism and Functions. Physiol. Revs, 63 (4) : 1420-1480.

Il est à noter que la lysine et la carnitine sous forme de chlorhydrate entrent dans la formulation de certaines compositions nutritionnelles, combinées à d'autres ingrédients actifs (Unlisted Drugs, volume 23, numéros 1 et 3, 1971).

Enfin, le document FR-M-4465 révèle un médicament destiné au traitement des asthénies et des anorexies comportant une association de chlorhydrate de DL-carnitine, de citrate de lysine tribasique et de vitamines C.

Au cours d'une recherche concernant les effets de la L-carnitine sur les fonctions musculaires, l'auteur de la présente invention a découvert, de façon tout à fait inattendue, que la L-lysine, ou ses sels d'acide organique ou inorganique, exerce un un effet de potentialisation ou de synergie vis-à-vis des effets biologiques de la L-carnitine.

Ainsi, la présente invention concerne plus particulièrement de nouvelles compositions pharmaceutiques et/ou diététiques caractérisées en ce qu'elles contiennent à titre de seul ingrédient actif de la L-carnitine, en combinaison avec de la L-lysine ou un de ses sels d'acide organique ou inorganique, en une quantité produisant un effet de potentialisation des effets biologiques de la L-carnitine.

Les compositions selon l'invention exercent des effets favorables au niveau du métabolisme azoté hydrocarboné ou électrolytique de divers tissus, et, par conséquent, au niveau des fonctions de ceux-ci dans différentes situations physiologiques ou pathologiques, notamment lorsque celles-ci sont accompagnées de perturbations des fonctions musculaires (hypoexcitabilité, hypocontractilité).

Ces compositions trouveront donc notamment application dans le traitement des états de dénutrition (en post-opératoire, post-infection, post-traumatisme, post-chimiothérapie, de l'insuffisance respiratoire chronique, du sujet âgé, du nouveau-né), des états prédiabétiques ou diabétiques, des hyperlipémiés ou encore dans le traitement des cardiopathies (angor, insuffisances cardiaques), de l'insuffisance hépatique, de la cirrhose. Ces compositions peuvent être également utilisées chez les hémodialysés.

Dans le domaine de la diététique, les compositions selon l'invention peuvent constituer un complément nutritionnel chez les sujets convalescents, les personnes âgées, le sportif occasionnel ou de haut niveau, la femme enceinte et, d'une manière générale, chez toute personne asthénique qui présente des perturbations fonctionnelles musculaires (hypoexcitabilité et hypocontractilité).

L'effet de synergie avec la L-carnitine est exercé par la L-lysine base, mais également par tous les sels d'acide organique ou inorganique de la L-lysine qui sont biologiquement acceptables. A titre d'exemples, on peut citer les sels de L-lysine suivants : chlorhydrate, acétate, succinate, $\alpha$-cétoglutarate, 3-hydroxybutyrate, 4-hydroxybutyrate, L-aspartate, L-glutamate, orotate.

Selon une caractéristique particulière de l'invention, la combinaison L-lysine et L-carnitine est une combinaison directe sous forme de sel.

On a pu observer en effet que l'effet de synergie avec la L-carnitine s'exerce également avec une combinaison directe sous forme de sel et notamment de chlorhydrate de L-carnitinate de L-lysine.

De tels sels peuvent être préparés selon des procédés traditionnels, par exemple par mélange de la L-carnitine avec la L-lysine sous forme de chlorhydrate dans de l'eau et cristallisation du sel résultant par addition d'un solvant polaire organique non miscible dans l'eau.

Dans les compositions selon l'invention, les proportions relatives de L-carnitine et de L-lysine peuvent varier entre 1:0,5 et 1:20 en moles.

Sous forme de combinaison avec la L-lysine, les doses par 24 h de L-carnitine se situent entre 0,5 et 3 g (soit 1,13 g à 6,78 g de la combinaison équimolaire de L-carnitine et de L-lysine, HCl), soit entre 1 et 6 g pour la combinaison équimolaire.

Les doses par 24 h de L-lysine se situent entre 0,5 g et 20 g. La dose de 20 g de L-lysine correspond à l'apport par 24 h de certains solutés d'acides aminés utilisés en nutrition artificielle parentérale.

L'invention concerne également l'utilisation de la L-lysine pour potentialiser l'effet de la L-carnitine dans la préparation d'un médicament destiné au traitement des perturbations musculaires liées à une hypoexcitabilité ou à une hypocontractilité.

La mise en évidence de l'effet de synergie exercé par la L-lysine (et ses sels) vis-à-vis des effets biologiques de la L-carnitine en combinaison équimolaire a été réalisée avec un modèle expérimental chez le Rat Wistar basé sur l'étude des fonctions musculaires (excitabilité, contractilité) perturbées par un jeûne de 5 jours.

Le choix de ce modèle a été guidé par le fait que les fonctions musculaires (excitabilité, contractilité) constituent un bon reflet de l'équilibre nutritionnel, métabolique et électrolytique de l'organisme. Des perturbations de cet équilibre sont observables dans des situations pathologiques variées, telles que les états de dénutrition, les troubles cardiovasculaires, les insuffisances respiratoires, le diabète, l'hémodialyse.

L'évaluation des fonctions neuromusculaires peut être réalisée à l'aide de méthodes électrophysiologiques : mesure de l'excitabilité neuromusculaire (LABORIT H. et LABORIT G., (1955), Excitabilité neuromusculaire et équilibre ionique. Intérêt pratique en chirurgie et en hibernothérapie. 1 vol., Masson éd., Paris), mesure de la contractilité musculaire (RUSSELL D. McR. et Coll. (1983) Skeletal muscle function during hypocaloric diets and fasting : A Comparison with standard nutritional assessment parameters. Am J. Clin. Nut., 37 : 133-138). Sur le plan pratique, ces méthodes offrent l'avantage d'être non invasives, répétitives, applicables à l'expérimentation animale et à l'homme en clinique.

Les conditions opératoires du modèle expérimental sont les suivantes :

## . Animaux et produits administrés

Des rats mâles Sprague-Dawley EOPS 0F1 (IFFA CREDO, France) ont été utilisés dans cette étude. Ils parviennent au Laboratoire à un poids moyen de 300 g et sont conservés par cage de dix pendant une semaine, avant toute manipulation. Ils reçoivent ad libidum de l'eau et une nourriture standard (U.A.R., France : protéines 17 %, lipides 3 %, glucides 59 %, cellulose, vitamines A et D3).

Les animaux sont ensuite répartis en lots distincts et isolés en cage individuelle. Ils reçoivent de l'eau ad libitum.

- Témoins nourris : animaux normalement nourris.
- Témoins à jeun I.P. : animaux soumis au jeûne pendant 5 jours consécutifs et recevant une administration quotidienne intrapéritonéale de soluté salé isotonique à 0,9 % (0,2 ml pour 100 g de poids corporel).
- Traités I.P. : animaux soumis au jeûne pendant 5 jours consécutifs et recevant une administration quotidienne intrapéritonéale de L-carnitine ou de L-lysine (HCl) ou de L-carnitine + L-lysine (HCl), dans un volume de 0,2 ml pour 100 g de poids corporel.
- Témoins à jeun P.O. : animaux soumis au jeune pendant 5 jours consécutifs et recevant une administration quotidienne par intubation gastrique de soluté salé isotonique à 0,9 % (0,2 ml pour 100 g de poids corporel).
- Traités P.O. : animaux soumis au jeûne pendant 5 jours consécutifs et recevant une administration quotidienne par intubation gastrique de L-carnitine ou de L-lysine, HCl ou de L-carnitine + L-lysine (HCl), dans un volume de 0,2 ml pour 100 g de poids corporel.

Les produits sont mis en solution dans du soluté salé isotonique à 0,9 %. La solution est ajustée à pH = 7 par une solution diluée d'acide chlorhydrique ou d'hydroxyde de sodium.

## . Préparation neuromusculaire (nerf sciatique - muscle gastrocnémien in situ)

La méthode d'étude de la réponse musculaire est basée sur celle décrite par Charlton M. P. et coll.(1981) sur la souris (Intracellular potassium activities in muscles of normal and dystrophic mice : an in vivo electrometric study. Experimental Neurology, 71 : 203-219), et reprise par Russell et coll. (1983), mentionnée ci-dessus et Russell D. McR. et coll. (1984) sur le rat (Metabolic and structural changes in skeletal muscle during hypocaloric dieting ; Am. J. Clin. Nutr., 39 : 503-513).

L'animal est anesthésié au pentobarbital sodique vétérinaire à la dose de 30 mg/kg, i.p. (0,05 ml pour 100 g de poids corporel). La veine fémorale droite est cathétérisée (catheter Abbocath, T 22G x 32 mm) et perfusée avec une solution diluée (1 %) de pentobarbital sodique vétérinaire. On utilise à cet effet un perfuseur automatique (B. Braun, Melsungen) à la vitesse de 0,380 ml/h.

L'animal est placé sur le flanc droit. La patte postérieure gauche est rasée. On pratique une incision de la peau parallèlement au fémur et au tibia jusqu'à la base du calcaneum. On sépare la peau des masses musculaires sous-jacentes, puis on pratique une incision de l'aponévrose dans les mêmes conditions que celles utilisées pour inciser la peau. On sépare les muscles en conservant leur vascularisation respective et on isole le tendon commun au muscle gastrocnémien et au muscle solaire.

On sépare le tendon propre au gastrocnémien, on le coupe à la base du calcanéum après l'avoir préalablement lié par un fil de soie. Celui-ci est relié à un capteur isotonique Electromed®, couplé à un enregistreur EJP 790® Electromed®.

La tension minimale exercée sur le muscle est étalonnée à 1 g. Le nerf sciatique est identifié et isolé sur 2 ou 3 cm de long et attaché par un fil de soie à 3 cm environ du gastrocnémien. Les branches latérales du nerf sciatique sont isolées et sectionnées. Une microélectrode de stimulation est appliquée sur le nerf sciatique à environ 1 cm du gastrocnémien.

L'animal est placé sur une planchette de liège, la patte postérieure gauche fixée au niveau du tibia à l'aide d'un pontet stabilisant la préparation. Il demeure au repos pendant 1 h dans une enceinte thermostatée qui maintient la température rectale de l'animal à 38°C, tout au long de la manipulation.

La préparation nerf-muscle est baignée d'une solution modifiée de Liley convenablement oxygénée (95 % $O_2$ et 5 % $CO_2$) à 37°C, (137 mM/l NaCl ; 5 mM/l KCl ; 2 mM/l $CaCl_2$ ; 1 mM/l $MgCl_2$ ; 24 mM/l $NaHCO_3$ ; 1 mM/l glutamate de sodium et 10 mM/l BES (acide N,N-bis[2-hydroxyéthyl]-2-aminoéthanesulfonique).

. Mesure de l'excitabilité neuro-musculaire

L'excitabilité neuro-musculaire est déterminée en mesurant 3 paramètres : la rhéobase, la chronaxie et le temps utile conformément aux méthodes décrites par LABORIT H. et LABORIT G., (1955), mentionnée ci-dessus et LAPICQUE L. (1926) "L'excitabilité en fonction du temps." 1 vol. Presses Universitaires éd., Paris.

La rhéobase est le seuil d'intensité électrique capable de provoquer le minimum de réponse fonctionnelle du muscle lorsque le temps de passage du courant s'allonge indéfiniment. Cette intensité minima demeure la même au-delà d'une certaine durée limite appelée temps utile. Si le temps de passage du courant devient inférieur au temps utile, l'intensité du courant capable de provoquer une réponse fonctionnelle devient de plus en plus forte. Pour un courant d'intensité double de la rhéobase, la durée la plus courte du passage du courant excitant est la chronaxie.

L'emploi d'un générateur d'impulsion rectangulaire de durée et d'intensité variables (ST Stimulator, JSI 0198) permet de tracer des courbes "intensité-durée" pour chaque animal. Comme la résistance de la préparation est supposée constante, on indiquera le voltage utilisé et non pas l'intensité. Le minimum de réponse fonctionnelle du muscle correspond à la force que celui-ci exerce pour entraîner un déplacement du stylet d'enregistrement de 1 mm. Elle est égale à 20 mg.

. Mesure de la contractilité musculaire

La contractilité musculaire est déterminée en mesurant la force de contraction provoquée par une stimulation de fréquence 10 Hz par rapport à la force de contraction provoquée par une stimulation de fréquence 100 Hz (force de contraction maxima).

La relation "force de contraction-fréquence de stimulation" du gastrocnémien est étudiée en appliquant une onde rectangulaire de stimulation dont la hauteur (voltage) est égale au double de la rhéobase et dont la largeur (temps) est égale au temps utile.

La relation entre la force de contraction musculaire et la fréquence de stimulation est étudiée en mesurant, deux fois consécutivement pour une fréquence donnée, l'amplitude de la contraction musculaire pour des fréquences de stimulation croissantes, délivrées pendant 2 s, une par minute de : 10, 20, 30, 40, 50 et 100 Hz.

. Choix de la dose de L-carnitine pour les études de synergie

Des essais préliminaires ont montré que la dose minimale active de L-carnitine par voie I.P. et par voie P.O., dans les conditions des essais, était de 77,5 μM/kg. Cette dose a été choisie pour la démonstration de l'effet de synergie des compositions selon l'invention, à base de L-carnitine.

. Durée de jeûne

Des essais préliminaires ont montré que les perturbations fonctionnelles musculaires n'apparaissent chez 100 % des animaux qu'au bout de 5 jours de jeûne.

RESULTATS :

Les résultats des mesures effectuées pour déterminer les effets comparés de la L-carnitine seule et de la L-carnitine en mélange équimolaire avec la L-lysine en solution aqueuse, sur l'excitabilité neuromusculaire et sur la contractilité musculaire du rat à jeun, sont respectivement donnés aux tableaux I (voie orale P.O.) et II (voie intrapéritonéale I.P.).

Ces résultats montrent que la combinaison équimolaire L-carnitine/L-lysine présente un effet maximum sur les paramètres étudiés dont les valeurs sont sensiblement ramenées au niveau de celles qui sont observées chez les témoins nourris.

On a également étudié les effets comparés de la L-carnitine combinée à des concentrations décroissantes de L-lysine sur l'excitabilité neuromusculaire et sur la contractilité musculaire du rat à jeun par voie orale.

Les résultats regroupés sur le tableau II A montrent que les proportions 1 : 0,5 et 1 : 0,25 de L-carnitine : L-lysine ont une activité inférieure à celle de la combinaison 1 : 1.

La proportion 1 : 0,25 n'a qu'un effet négligeable tandis que l'effet est plus marqué pour la proportion 1 : 0,5.

Par ailleurs, les résultats regroupés sur le tableau II B montrent que, par rapport au mélange 1 : 1, un léger gain apparaît pour le mélange L-carnitine : L-lysine 1 : 2 qui n'est pas amélioré par les proportions 1 : 4 et 1 : 8.

On peut donc considérer que l'optimum se situe au voisinage des proportions 1 : 1 à 1 : 2.

Dans les mêmes conditions expérimentales, on a étudié l'effet de la L-lysine seule et les résultats représentés au tableau III montrent que la L-lysine seule, par voie orale, n'a pas ou n'a que peu d'effet. L'absence d'effet est totale à la dose de 77,5 µM/kg pour laquelle l'effet de synergie vis-à-vis de la L-carnitine a été observé.

L'effet demeure faible et non significatif pour des doses respectivement 2, 4 et 8 fois plus élevées.

Ainsi, la L-lysine seule n'exerce pas l'activité manifestée par la combinaison L-carnitine + L-lysine. La combinaison L-carnitine + L-lysine présente donc une activité accrue (effet de potentialisation ou de synergie) qui peut être expliquée par une augmentation de biodisponibilité de la L-carnitine, au niveau des organes-cibles, muscles et myocarde.

L'augmentation de biodisponibilité de la L-carnitine résulterait en premier lieu de la synthèse endogène de celle-ci à partir de la L-lysine, qui joue ainsi le rôle de prodrogue.

Parallèlement à la diminution des doses de L-carnitine à administrer pour une activité équivalente, le mécanisme de synthèse endogène permet de diminuer les pics plasmatiques de L-carnitine, ce qui réduit les risques de perte urinaire, tout en assurant un meilleur rendement de la captation tissulaire de la L-carnitine par les muscles et par le myocarde grâce à une cinétique de distribution de type instantané-retard.

De ce fait également, les risques d'apparition d'effets indésirables du produit par voie orale (épisodes diarrhéiques) sont diminués.

## TABLEAU I

Effets comparés de la L-carnitine seule et de la L-carnitine associée à la L-lysine sur l'excitabilité neuromusculaire et sur la contractilité musculaire du rat à jeun.
(voie P. O.)

| P.O. | EXCITABILITE NEUROMUSCULAIRE | | | CONTRACTILITE MUSCULAIRE |
|---|---|---|---|---|
| | RHEOBASE (Volt) | CHRONAXIE (ms x $10^{-4}$) | TEMPS UTILE (ms x $10^{-4}$) | AMPLITUDE A 10 Hz / AMPLITUDE A 100 Hz (%) |
| Témoins nourris n = 8 | 0,30 + 0,03 | 197 + 16 | 488 + 13 | 12,81 + 0,62 |
| Témoins à jeun n = 8 | 0,81 + 0,03 aaa | 257 + 7 aa | 575 + 16 aaa | 19,71 + 1,15 aaa |
| Traités L-carnitine 77,5 µM/kg n = 8 | 0,63 + 0,05 b | 247 + 10 NSb | 569 + 13 NSb | 18,45 + 1,1 NSb |
| Traités L-carnitine + L-lysine, HCl 77,5 µM/kg n = 8 | 0,31 + 0,01 bbb | 219 + 13 b | 519 + 13 b | 14,72 + 0,59 bb |

a = comparaison avec témoins nourris    a ou b    : 0,01 < P < 0,1      aa ou bb    : 0,001 < P < 0,02
b = comparaison avec témoins à jeun    aaa ou bbb : P < 0,001      NSa ou NSb : non significatif

EP 0 354 848 B1

## TABLEAU II

Effets comparés de la L-carnitine seule et de la L-carnitine associée à la L-Lysine sur l'excitabilité neuromusculaire et sur la contractilité musculaire du rat à jeun (voie I.P.)

| I.P. | EXCITABILITE NEUROMUSCULAIRE | | | CONTRACTILITE MUSCULAIRE |
|---|---|---|---|---|
| | RHEOBASE (Volt) | CHRONAXIE (ms x $10^{-4}$) | TEMPS UTILE (ms x $10^{-4}$) | AMPLITUDE A 10 Hz / AMPLITUDE A 100 Hz (%) |
| Témoins nourris n = 8 | $0,30 \pm 0,03$ | $197 \pm 16$ | $488 \pm 13$ | $12,81 \pm 0,62$ |
| Témoins à jeun n = 8 | $0,87 \pm 0,07$ aaa | $273 \pm 10$ aa | $588 \pm 23$ aa | $17,92 \pm 0,43$ aaa |
| Traités L-carnitine 77,5 µM/kg + NaCl 0,9 % n = 8 | $0,65 \pm 0,09$ NSb | $247 \pm 15$ NSb | $588 \pm 30$ NSb | $17,80 \pm 0,82$ NSb |
| Traités L-carnitine + L-Lysine, HCl 77,5 µM/kg a/a n = 8 | $0,31 \pm 0,01$ bbb | $196 \pm 10$ bbb | $488 \pm 23$ bb | $12,14 \pm 0,63$ bbb |

a : comparaison avec témoins nourris          a ou b : $0,01 < P < 0,1$          aa ou bb = $0,001 < P < 0,02$

b : comparaison avec témoins à jeun          aaa ou bbb = $P < 0,001$          NSa ou NSb = non significatif

EP 0 354 848 B1

TABLEAU II A

Action comparée de la L-carnitine, combinée à des concentrations décroissantes de L-lysine, sur l'excitabilité neuromusculaire et sur la contractilité musculaire du rat à jeun. (voie P. O.)

| P.O. | EXCITABILITE NEUROMUSCULAIRE | | | CONTRACTILITE MUSCULAIRE |
|---|---|---|---|---|
| | RHEOBASE (Volt) | CHRONAXIE (ms x $10^{-4}$) | TEMPS UTILE (ms x $10^{-4}$) | AMPLITUDE A 10 Hz / AMPLITUDE A 100 Hz (%) |
| Témoins nourris n = 8 | 0,30 ± 0,03 | 197 ± 16 | 488 ± 13 | 12,81 ± 0,62 |
| Témoins à jeun n = 8 | 0,70 ± 0,06 aaa | 258 ± 9 aa | 581 ± 13 aaa | 18,85 ± 1,80 aa |
| L-carnitine 77,5 µM/kg n = 8 | 0,61 ± 0,04 NSb | 247 ± 10 NSb | 550 ± 27 NSb | 17,63 ± 1,30 NSb |
| L-carnitine 77,5 µM/kg + L-lysine 19,375 µM/kg (combinaison 1:0,25) n = 8 | 0,52 ± 0,04 b ccc | 249 ± 12 NSb c | 556 ± 14 NSb NSc | 17,40 ± 1,45 NSb NSc |
| L-carnitine 77,5 µM/kg + L-lysine 38,75 µM/kg (combinaison 1:0,5) n = 8 | 0,43 ± 0,04 bb cc | 233 ± 8 NSb NSc | 550 ± 16 NSb NSc | 18,20 ± 1,40 NSb c |
| L-carnitine 77,5 µM/kg + L-lysine 77,5 µM/kg (combinaison 1:1) n = 8 | 0,30 ± 0,01 bbb | 215 ± 8 bb | 507 ± 23 b | 14,00 ± 1,20 b |

a = comparaison avec témoins nourris ; a : 0,01 < P < 0,1  aa : 0,001 < P < 0,02  aaa : P < 0,001 ; NSa : non significatif.

b = comparaison avec témoins à jeun  ;  b, bb, bbb, NSb : cf a.

c = comparaison avec L-carnitine + L-lysine 77,5 µM/kg (1:1). c, cc, ccc, NSc : cf a.

TABLEAU II B

Action comparée de la L-carnitine combinée à des concentrations croissantes de L-lysine sur l'excitabilité et la contractilité musculaire du rat à jeun (voie Per OS)

| P.O. | EXCITABILITE NEUROMUSCULAIRE | | | CONTRACTILITE MUSCULAIRE |
|---|---|---|---|---|
| | RHEOBASE (Volt) | CHRONAXIE (ms x $10^{-4}$) | TEMPS UTILE (ms x $10^{-4}$) | AMPLITUDE A 10 Hz / AMPLITUDE A 100 Hz (%) |
| L-carnitine 38,75 µM/kg et L-lysine Hcl 38,75 µM/kg Mélange 1:1 N = 8 | 0,56 + 0,08 | 243 + 12 | 568 + 16 | 17,22 + 0,72 |
| L-carnitine 38,75 µM/kg et L-lysine Hcl 77,5 µM/kg Mélange 1 : 4 n = 8 | 0,44 + 0,04 NSa | 230 + 13 NSa | 550 + 16 NSa | 16,46 + 1,36 NSa |
| L-carnitine 38,75 µM/kg et L-lysine 155 µM/kg Mélange 1 : 4 n = 7 | 0,46 + 0,06 NSa | 246 + 12 NSa | 535 + 14 NSa | 16,02 + 0,49 NSa |
| L-carnitine 38,75 µM/kg et L-lysine Hcl 310 µM/kg Mélange 1 : 8 n = 8 | 0,49 + 0,05 NSa | 247 + 10 NSa | 543 + 17 NSa | 16,13 + 0,88 NSa |

a : comparaison avec le groupe recevant le mélange 1 : 1     NSa : non significatif

## TABLEAU III

Effet de la L-lysine seule sur l'excitabilité neuromusculaire et sur la contractilité musculaire du rat à jeun (voie P.O.)

| P.O. | EXCITABILITE NEUROMUSCULAIRE | | | CONTRACTILITE MUSCULAIRE |
|---|---|---|---|---|
| | RHEOBASE (Volt) | CHRONAXIE (ms x $10^{-4}$) | TEMPS UTILE (ms x $10^{-4}$) | AMPLITUDE A 10 Hz / AMPLITUDE A 100 Hz (%) |
| Témoins nourris n = 8 | 0,30 + 0,03 | 197 + 16 | 488 + 13 | 12,81 + 0,62 |
| Témoins à jeun n = 7 | 0,74 + 0,04 aa | 261 + 9 aa | 586 + 9 aa | 17,72 + 0,66 aaa |
| Traités L-lysine HCl 77,5 µM/kg n = 7 | 0,63 + 0,04 NSb | 266 + 10 NSb | 571 + 15 NSb | 16,80 + 1,33 NSb |
| 155 µM/kg n = 7 | 0,56 + 0,04 b | 249 + 12 NSb | 557 + 17 NSb | 16,16 + 0,99 NSb |
| 310 µM/kg n = 7 | 0,49 + 0,07 b | 245 + 17 NSb | 557 + 20 NSb | 16,46 + 0,99 NSb |
| 620 µM/kg n = 7 | 0,52 + 0,06 b | 243 + 20 NSb | 550 + 16 NSb | 15,96 + 0,65 NSb |

a : comparaison avec témoins nourris    a ou b    : $0,01 < P < 0,1$    aa ou bb    : $0,001 < P < 0,02$

b : comparaison avec témoins à jeun    aaa ou bbb : $P < 0,001$    NSa ou NSb : non significatif

EP 0 354 848 B1

On a donné au tableau IV les résultats d'une étude effet-dose d'une combinaison équimolaire de L-carnitine et de L-lysine sur l'excitabilité et la contractilité musculaire du rat à jeun (voie P.O.).

Dans les conditions expérimentales, la dose minimale active est voisine de 20 µM/kg. Le maximum d'activité est atteint à la dose de 77,5 µM/kg.

La dose efficace 50 % (DE50 = dose qui induit une correction de 50 % de la différence entre les valeurs des témoins à jeûn et des témoins nourris) est voisine de 30 µM/kg pour la combinaison équimolaire de L-carnitine et de L-lysine.

Elle est voisine de 100 µM/kg pour la L-carnitine seule.

A activité égale, les doses (µM/kg) du mélange équimolaire de L-carnitine et de L-lysine sont environ 3 fois moindres que celles de la L-carnitine seule. Le gain de synergie est donc de 3. On observe le même gain de synergie avec la forme sel de L-carnitine et de L-lysine.

En conséquence, à bénéfices biologiques équivalents, les combinaisons selon l'invention de L-carnitine et de L-lysine (ou ses sels) correspondent à des quantités de L-carnitine nettement inférieures à celles qui sont nécessaires avec la L-carnitine-seule pour obtenir les mêmes effets.

Les compositions selon l'invention présentent donc un interêt évident, non seulement du point de vue économique, mais également en ce qui concerne les doses de L-carnitine à absorber.

## T A B L E A U  I V

Etude effet-dose d'une combinaison équimolaire de L-carnitine et de L-lysine sur l'excitabilité et la contractilité musculaires du rat à jeun (voie P.O.)

| P.O. | EXCITABILITE NEUROMUSCULAIRE | | | CONTRACTILITE MUSCULAIRE |
|---|---|---|---|---|
| | RHEOBASE (Volt) | CHRONAXIE (ms x $10^{-4}$) | TEMPS UTILE (ms x $10^{-4}$) | $\dfrac{\text{AMPLITUDE A 10 Hz}}{\text{AMPLITUDE A 100 Hz}}$ (%) |
| Témoins nourris n = 8 | 0,30 + 0,03 | 197 + 16 | 488 + 13 | 12,81 + 0,62 |
| Témoins à jeun n = 8 | 0,86 + 0,04 aaa | 272 + 10 aa | 587 + 8 aaa | 18,57 + 0,88 aaa |
| Traités L-carnitine + L-Lysine, HCl | | | | |
| . 19,375 µM/kg n = 8 | 0,73 + 0,04 b | 269 + 6 NSb | 581 + 13 NSb | 17,68 + 0,65 NSb |
| . 38,75 µM/kg n = 8 | 0,44 + 0,06 bbb | 231 + 9 b | 550 + 16 NSb | 14,25 + 1,25 b |
| . 77,5 µM/kg n = 8 | 0,39 + 0,06 bbb | 218 + 7 bbb | 537 + 15 b | 12,33 + 0,75 bbb |
| . 155 µm/kg n = 8 | 0,31 + 0,01 bbb | 206 + 4 bbb | 518 + 13 bbb | 12,98 + 0,54 bbb |

a : comparaison avec témoins nourris    a ou b    : 0,01 < P < 0,1     aa ou bb    : 0,001 < P < 0,02

b : comparaison avec témoins à jeun    aaa ou bbb : P < 0,001     NSa ou NSb : non significatif

Les tableaux V et VI représentent les effets comparés de la L-carnitine associée à la L-lysine et respectivement de la L-carnitine associée en mélange en solution aqueuse à la L-arginine (tableau V), à la L-ornithine ou à l'acide aspartique (tableau VI).

Les résultats indiquent clairement que la L-lysine exerce vis-à-vis de l'activité de la L-carnitine un effet de synergie spécifique qui la distingue :

- d'une part, des autres composés biochimiques réputés pour leur activité au niveau musculaire, tels que l'acide aspartique ;

- d'autre part, des composés structurellement proches de la lysine, en particulier l'arginine et l'ornithine.

Ces tableaux démontrent par conséquent le caractère imprévisible et inattendu de l'effet de synergie observé, qui est à la base de la présente invention.

Il est enfin à noter que l'activité de la combinaison L-carnitine L-lysine est très largement supérieure à celle d'une combinaison DL-carnitine : L-lysine.

Ceci résulte notamment des résultats reportés au tableau VII d'une étude de l'action comparée des combinaisons de L-carnitine : ou de DL-carnitine avec la L-lysine sur l'excitabilité neuromusculaire et sur la contractilité musculaire du rat à jeun (par voie orale).

EP 0 354 848 B1

## TABLEAU V

Effets comparés de la L-carnitine associée à la L-Lysine ou à la L-arginine sur l'excitabilité neuromusculaire et sur la contractilité musculaire du rat à jeun (voie I.P.)

| I.P. | EXCITABILITE NEUROMUSCULAIRE | | | CONTRACTILITE MUSCULAIRE |
|---|---|---|---|---|
| | RHEOBASE (Volt) | CHRONAXIE (ms X $10^{-4}$) | TEMPS UTILE (ms x $10^{-4}$) | $\dfrac{\text{AMPLITUDE A 10 Hz}}{\text{AMPLITUDE A 100 Hz}}$ (%) |
| Témoins nourris n = 8 | 0,30 ± 0,03 | 197 ± 16 | 488 ± 13 | 12,81 ± 0,62 |
| Témoins à jeun n = 8 | 0,87 ± 0,07 aaa | 273 ± 10 aa | 588 ± 23 aa | 17,92 ± 0,43 aaa |
| Traités L-carnitine 77,5 µM/kg + NaCl 0,9 % n = 8 | 0,60 ± 0,09 NSb | 213 ± 16 b | 617 ± 31 NSb | 15,39 ± 1,28 NSb |
| Traités L-carnitine + L-Lysine, HCl 77,5 µM/kg a/a n = 8 | 0,31 ± 0,01 bbb | 196 ± 10 bbb | 488 ± 23 bb | 12,14 ± 0,63 bbb |
| Traités L-carnitine + L-arginine, HCl 77,5 µm/kg a/a n = 8 | 0,33 ± 0,04 bbb | 244 ± 15 NSb | 563 ± 32 NSb | 14,74 ± 0,87 bb |

a : comparaison avec témoins nourris      a ou b    : 0,01 < P < 0,1 ;      aa ou bb    : 0,001 < P < 0,02

b : comparaison avec témoins à jeun      aaa ou bbb : P < 0,001 ;      NSa ou NSb : non significatif

## TABLEAU VI

Effets comparés de la L-carnitine associée à la L-lysine ou à la L-ornithine ou à l'acide aspartique sur l'excitabilité neuromusculaire et sur la contractilité musculaire du rat à jeun (voie I.P.)

| I.P. | EXCITABILITE NEUROMUSCULAIRE | | | CONTRACTILITE MUSCULAIRE |
|---|---|---|---|---|
| | RHEOBASE (Volt) | CHRONAXIE $(ms \times 10^{-4})$ | TEMPS UTILE $(ms \times 10^{-4})$ | AMPLITUDE A 10 Hz / AMPLITUDE A 100 Hz (%) |
| Témoins nourris n = 8 | 0,30 + 0,03 | 197 + 16 | 488 + 13 | 12,81 + 0,62 |
| Témoins à jeun n = 8 | 0,81 + 0,06 aaa | 260 + 13 aa | 571 + 15 aa | 18,53 + 0,42 aaa |
| Traités L-carnitine 77,5 µM/kg + NaCl 0,9 % n = 8 | 0,61 + 0,04 NSb | 247 + 10 NSb | 550 + 27 NSb | 17,63 + 1,27 NSb |
| Traités L-carnitine + L-lysine, HCl 77,5 µM/kg a/a n = 8 | 0,39 + 0,04 bbb | 209 + 10 bb | 488 + 23 bb | 13,20 + 0,48 bbb |
| Traités L-carnitine + L-ornithine, HCl 77,5 µM/kg a/a n = 8 | 0,60 + 0,03 NSb | 240 + 11 NSb | 550 + 16 NSb | 16,75 + 0,77 NSb |
| Traités L-carnitine + acide L-aspartique 77,5 µM/kg a/a n = 8 | 0,41 + 0,05 bbb | 232 + 14 NSb | 531 + 16 NSb | 15,80 + 1,38 NSb |

a : comparaison avec témoins nourris    a ou b   : 0,01 < P < 0,1    aa ou bb   : 0,001 < P < 0,02
b : comparaison avec témoins à jeun    aaa ou bbb : P < 0,001 ;    NSa ou NSb : non significatif

EP 0 354 848 B1

On donnera ci-après divers exemples de formulations de compositions pharmaceutiques et/ou diététiques que l'on peut réaliser selon l'invention.

D'une façon générale, les combinaisons de L-carnitine et de L-lysine selon l'invention peuvent être présentées sous des formes variées destinées à l'usage pharmaceutique ou diététique.

Les présentations peuvent être destinées :

-à la voie orale : solutions buvables (ampoules, flacons, pack, poches), gouttes, sirops, sachets, lyophili-

## TABLEAU VII

Action comparée des combinaisons de L-carnitine ou de DL-carnitine avec la L-lysine sur l'excitabilité neuromusculaire et sur la contractilité musculaire du rat à jeûn (voie P. O.)

| P.O. | EXCITABILITE NEUROMUSCULAIRE | | | CONTRACTILITE MUSCULAIRE |
|---|---|---|---|---|
| | RHEOBASE (Volt) | CHRONAXIE (ms x $10^{-4}$) | TEMPS UTILE (ms x $10^{-4}$) | AMPLITUDE A 10 Hz / AMPLITUDE A 100 Hz (%) |
| Témoins nourris n = 8 | $0,30 \pm 0,03$ | $197 \pm 16$ | $488 \pm 13$ | $12,81 \pm 0,62$ |
| Témoins à jeun n = 8 | $0,84 \pm 0,05$ aaa | $267 \pm 11$ aaa | $587 \pm 8$ aaa | $20,15 \pm 0,45$ aaa |
| DL-carnitine + L-lysine 38,75 µM/kg (1:1) n = 8 | $0,66 \pm 0,09$ NSb c | $264 \pm 11$ NSb c | $563 \pm 18$ NSb NSc | $18,93 \pm 0,95$ NSb c |
| L-carnitine + L-lysine 38,75 µM/kg (1:1) n = 8 | $0,44 \pm 0,04$ bbb c | $227 \pm 9$ b c | $544 \pm 15$ b NSc | $15,81 \pm 0,90$ bbb c |
| DL-carnitine + L-lysine 77,50 µM/kg (1:1) n = 8 | $0,49 \pm 0,02$ bbb ddd | $229 \pm 9$ b NSd | $544 \pm 17$ b NSd | $16,52 \pm 0,70$ bbb d |
| L-carnitine + L-lysine 77,50 µM/kg (1:1) n = 8 | $0,33 \pm 02$ bbb ddd | $216 \pm 8$ bb NSd | $519 \pm 9$ bbb NSd | $13,07 \pm 1,10$ bbb d |

a : comparaison avec témoins nourris ;  a : $0,01 < P < 0,1$  aa : $0,001 < P < 0,02$  aaa : $P < 0,001$  NSa : non significatif.

b : comparaison avec témoins à jeun ; b, bb, bbb, NSb : cf a.

c : comparaison entre DL-carnitine + L-lysine et L-carnitine + L-lysine (38,75 µM/kg) ; c, cc, ccc, NSc : cf a.

d : identique à c pour la dose de 77,50 µM/kg ; d, dd, ddd, NSd : cf a.

sats oraux (lyocs), gélules, comprimés, tablettes à mâcher, boissons reconstituantes.

-à la voie injectable intraveineuse :

.soit sous forme de soluté injectable administré avec un solution de perfusion (glucosé, salé) ou avec des solutés nutritifs,

.soit sous forme de solutés de nutrition artificielle parentérale (glucides, acides aminés, peptides, lipides, électrolytes, oligo-éléments, vitamines).

-à la voie entérale, en association avec les préparations pour alimentation artificielle entérale.

Exemple 1

**FORME INJECTABLE**

| | Solution à 10 % | Solution à 20 % |
|---|---|---|
| L-carnitine base ............... | 0,500 g | 1,0 g |
| L-lysine chlorhydrate .......... | 0,565 g | 1,13 g |
| HCl q.s.p. ..................... | pH 4,5 | pH 4,5 |
| Eau p.p.i. q.s.p. .............. | 10, - ml | 10, - ml |

Pour utilisation à travers un soluté de perfusion.

**FORMES ORALES**

Exemple 2 : Ampoules buvables

| | Solution à 20 % | Solution à 10 % |
|---|---|---|
| L-carnitine base ............... | 0,500 g | 0,500 g |
| L-lysine chlorhydrate .......... | 0,566 g | 0,566 g |
| HCl q.s.p. ..................... | pH 4,5 | pH 4,5 |
| Saccharinate Na ................ | 0,003 g | 0,005 g |
| Arôme orange ................... | 0,020 g | 0,040 g |
| Eau purifiée q.s.p. ............ | 5, - ml | 10, - ml |

Exemple 3 : Gouttes

| | Solution à 30 % |
|---|---|
| L-carnitine base ............... | 15, - g |
| L-lysine chlorhydrate .......... | 16,98 g |
| HCl q.s.p. ..................... | pH 4,5 |
| Benzoate sodium ................ | 0,10 g |
| Eau purifiée q.s.p. ............ | 100, - ml |

Flacon de 90 ml avec mesure de 2 ml (640 mg).

Exemple 4 : Sachet

| | |
|---|---|
| L-carnitine base ............... | 0,5   g |
| L-lysine chlorhydrate .......... | 0,565 g |
| Sucre (saccharose) ............. | 3,920 g |
| Essence citron ................. | 0,015 g |
| | 5,-   g |

Exemple 5 : Lyophilisat oral

| | |
|---|---|
| L-carnitine base ............... | 0,250 g |
| L-lysine chlorhydrate .......... | 0,283 g |
| Dextran 70 ..................... | 0,005 g |
| Gomme XANTHANE ................. | 0,005 g |
| Arôme orange ................... | 0,020 g |
| Acide citrique ................. | 0,005 g |
| Saccharinate Na ................ | 0,003 g |
| Mannitol ....................... | 0,429 g |
| Eau ............................ | 0,500 g |
| | 1,500 g |

Exemple 6 : Tablette à mâcher (chewable)

| | |
|---|---|
| L-carnitine base ............... | 0,250  g |
| L-lysine chlorhydrate .......... | 0,283  g |
| Maltodextrine .................. | 0,500  g |
| Saccharose ..................... | 0,327  g |
| Levulose ....................... | 0,300  g |
| Acide citrique ................. | 0,045  g |
| Citrate potassium .............. | 0,0075 g |
| Hydroxyde de magnésie .......... | 0,0050 g |
| Arôme orange ................... | 0,0025 g |
| | 2,220  g |

Exemple 7 : Formulation pour boisson reconstituante

```
L-carnitine base ...............      0,250 g
L-lysine chlorhydrate ..........      0,283 g
Saccharose .....................      9,717 g
Maltodextrine ..................      1,200 g
Levulose .......................      1, -  g
Glucose ........................      1, -  g
Acide citrique .................      0,800 g
Citrate potassium ..............      0,250 g
Acide ascorbique ...............      0,250 g
Arôme citron ...................      0,200 g
Chlorure sodium ................      0,050 g
                                     ─────────
                                     15,-   g
```

Boisson à reconstituer par dissolution dans 180 ml d'eau potable fraîche.

L'invention concerne aussi un procédé de préparation d'une composition pharmaceutique et/ou diététique, caractérisé en ce qu'on combine la L-carnitine avec la L-lysine ou un de ses sels d'acide organique ou inorganique et en ce que l'on mélange cette combinaison à des excipients pharmaceutiquement ou diététiquement acceptables. Les modes de réalisation particuliers sont conformes à ceux précédemment décrits pour la composition.

L'invention concerne enfin un procédé de traitement de mammifères, en particulier un être humain ou un animal, caractérisé en ce qu'on administre au mammifère une combinaison de L-carnitine et de L-lysine ou un de ses sels d'acide organique ou inorganique.

De préférence, on traite des situations physiologiques ou pathologiques notamment des perturbations musculaires accompagnées d'une hypoexcitabilité et/ou d'une hypocontractilité musculaire, comme par exemple les états de dénutrition, les états prédiabétiques ou diabétiques, dans l'hémodyalyse, les hyperlipémies, les cardiopathies et les insuffisances hépatiques.

## Revendications

**Revendications pour les Etats contractants suivants : DE, GB, FR, IT, NL, SE, CH, LI, BE, AT, LU**

1. Compositions pharmaceutiqques et/ou diététiques, caractérisées en ce qu'elles contiennent à titre de seul ingrédient actif de la L-carnitine, en combinaison avec de la L-lysine ou un de ses sels d'acide organique ou inorganique, en une quantité produisant un effet de potentialisation des effets biologiques de la L-carnitine.

2. Compositions selon la revendication 1, caractérisées en ce que la combinaison précitée est formée par le mélange en solution aqueuse de L-carnitine et de L-lysine ou un de ses sels d'acide organique ou inorganique.

3. Compositions selon la revendication 1, caractérisées en ce que la combinaison précitée est formée par un sel de L-carnitine et de L-lysine, par exemple le chlorhydrate de L-carnitinate de L-lysine.

4. Compositions selon l'une des revendications 1 à 3, caractérisées en ce que les proportions relatives de L-carnitine et de L-lysine sont respectivement comprises entre 1:0,5 et 1:20 en moles.

5. Compositions selon l'une quelconque des revendications 1 à 4, caractérisées en ce que la proportion relative de L-carnitine et de L-lysine est équimolaire.

6. Compositions selon l'une quelconque des revendications 1 à 5, caractérisées en ce qu'elles sont présentes sous forme compatible avec une administration orale ou injectable, à une posologie quotidienne de 1 à 6 g de la combinaison équimolaire de L-carnitine et de L-lysine.

7. Compositions selon l'une quelconque des revendications 1 à 6, caractérisées en ce qu'elles sont sous forme injectable et en ce qu'elles comprennent :

| | |
|---|---|
| L-Carnitine base | 0,500 g |
| L-lysine Chlorhydrate | 0,565 g |
| HCl q.s.p. | pH 4,5 |
| Eau p.p.i. q.s.p. | 5 ou 10, -ml |

Pour utilisation à travers un soluté de perfusion.

8. Compositions selon l'une quelconque des revendications 1 à 6, caractérisées en ce qu'elles sont sous formes orales et en ce qu'elles comprennent :

| | |
|---|---|
| L-carnitine base | 0,500 g |
| L-lysine Chlorhydrate | 0,566 g |
| HCl q.s.p. | pH 4,5 |
| Saccharinate Na | 0,003 g |
| Arôme orange | 0,020 g |
| Eau purifiée q.s.p. | 5, -ml |

9. Compositions selon l'une quelconque des revendications 1 à 6, caractérisées en ce qu'elles sont sous forme de gouttes et en ce qu'elles comprennent :

| | |
|---|---|
| L-carnitine base | 15, - g |
| L-lysine chlorhydrate | 16,98 g |
| HCl q.s.p. | pH 4,5 |
| Benzoate sodium | 0,10 g |
| Eau purifiée q.s.p. | 100, -ml |

Flacon de 90 ml avec mesure de 2 ml (640 mg).

10. Compositions selon l'une quelconque des revendications 1 à 6, caractérisées en ce qu'elles sont sous forme de sachet et en ce qu'elles comprennent :

| | |
|---|---|
| L-carnitine base | 0,5 g |
| L-lysine chlorhydrate | 0,565 g |
| Sucre (saccharose) | 3,920 g |
| Essence Citron | 0,015 g |

11. Compositions selon l'une quelconque des revendications 1 à 6, caractérisées en ce qu'elles sont sous forme de lyophilisat oral et en ce qu'elles comprennent :

| | |
|---|---|
| L-carnitine base | 0,250 g |
| L-lysine chlorhydrate | 0,283 g |
| Dextran 70 | 0,005 g |
| Gomme XANTHANE | 0,005 g |
| Arôme orange | 0,020 g |
| Acide Citrique | 0,005 g |
| Saccharinate Na | 0,003 g |
| Mannitol | 0,429 g |
| Eau | 0,500 g |

12. Compositions selon l'une quelconque des revendications 1 à 6, caractérisées en ce qu'elles sont sous forme de tablette à mâcher (chewable) et en ce qu'elles comprennent :

| | |
|---|---|
| L-carnitine base | 0,250 g |
| L-lysine chlorhydrate | 0,283 g |
| Maltodextrine | 0,500 g |
| Saccharose | 0,327 g |
| Levulose | 0,800 g |
| Acide citrique | 0,045 g |

| Citrate potassium | 0,0075 g |
|---|---|
| Hydroxyde de magnésie | 0,0050 g |
| Arôme orange | 0,0025 g |

**13.** Procédé de préparation d'une composition pharmaceutique et/ou diététique selon l'une des revendications 1 à 12, caractérisé en ce que l'on combine la L-carnitine avec la L-lysine ou un de ses sels d'acide organique ou inorganique et en ce que l'on mélange cette combinaison à des excipients pharmaceutiquement et/ou diététiquement acceptables.

**14.** Utilisation de la L-lysine pour potentialiser l'effet de la L-carnitine dans la préparation d'un médicament destiné au traitement des perturbations musculaires liées à une hypoexcitabilité ou à une hypocontractilité.

**Revendications pour les Etats contractants suivants : ES, GR**

**1.** Procédé de préparation d'une composition pharmaceutique et/ou diététique, caractérisé en ce qu'il consiste à combiner la L-carnitine avec la L-lysine ou un de ses sels d'acide organique ou inorganique, en une quantité produisant un effet de potentialisation des effets biologiques de la L-carnitine, et à mélanger cette combinaison à des excipients et/ou diététiquement acceptables.

**2.** Procédé selon la revendication 1, caractérisé en ce qu'il comprend le mélange en solution aqueuse de la L-carnitine et de la L-lysine ou d'un de ses sels d'acide organique ou inorganique.

**3.** Procédé selon la revendication 1, caractérisé en ce qu'il comprend la formation d'un sel de L-carnitine et de L-lysine, par exemple le chlorhydrate de L-carnitinate de L-lysine.

**4.** Procédé selon l'une des revendications 1 à 3, caractérisé en ce que la L-carnitine et la L-lysine sont combinées dans des proportions relatives comprises entre 1:0,5 et 1:20, exprimées en moles.

**5.** Procédé selon l'une des revendications 1 à 4, caractérisé en ce que la L-carnitine et la L-lysine sont combinées dans des proportions équimolaires.

**6.** Procédé selon l'une des revendications 1 à 5, pour la préparation d'une forme compatible avec une administration orale ou injectable, à une posologie quotidienne de 1 à 6 g de la combinaison équimolaire de L-carnitine et de L-lysine.

**7.** Procédé selon l'une des revendications 1 à 6, pour la préparation d'une forme injectable comprenant :

| L-carnitine base | 0,500 g |
|---|---|
| L-lysine chlorhydrate | 0,565 g |
| HCl q.s.p. | pH 4,5 |
| Eau p.p.i. q.s.p. | 5 ou 10,- ml |

Pour utilisation à travers un soluté de perfusion.

**8.** Procédé selon l'une des revendications 1 à 6, pour la préparation de formes orales, comprenant :

| L-carnitine base | 0,500 g |
|---|---|
| L-lysine chlorhydrate | 0,566 g |
| HCl q.s.p | pH 4,5 |
| Saccharinate Na | 0,003 g |
| Arôme orange | 0,020 g |
| Eau purifiée q.s.p. | 5,- ml |

**9.** Procédé selon l'une des revendications 1 à 6, pour la préparation d'une composition sous forme de gouttes comprenant :

| L-carnitine base | 15,- g |
|---|---|
| L-lysine chlorhydrate | 16,98 g |
| HCl q.s.p. | pH 4,5 |
| Benzoate sodium | 0,10 g |
| Eau purifiée q.s.p. | 100,- ml |

Flacon de 90 ml avec mesure de 2 ml (640 mg).

**10.** Procédé selon l'une quelconque des revendications 1 à 6, pour la préparation de sachets comprenant :

| | |
|---|---|
| L-carnitine base | 0,5 g |
| L-lysine chlorhydrate | 0,565 g |
| Sucre (saccharose) | 3,920 g |
| Essence citron | 0,015 g |

**11.** Procédé selon l'une quelconque des revendications 1 à 6, pour la préparation d'un lyophilisat orale comprenant :

| | |
|---|---|
| L-carnitine base | 0,250 g |
| L-lysine chlorhydrate | 0,283 g |
| Dextran 70 | 0,005 g |
| Gomme XANTHANE | 0,005 g |
| Arôme orange | 0,020 g |
| Acide citrique | 0,005 g |
| Saccharinate Na | 0,003 g |
| Mannitol | 0,429 g |
| Eau | 0,500 g |

**12.** Procédé selon l'une quelconque des revendications 1 à 6, pour la préparation d'une tablette à macher comprenant :

| | |
|---|---|
| L-carnitine base | 0,250 g |
| L-lysine chlorhydrate | 0,283 g |
| Maltodextrine | 0,500 g |
| Saccharose | 0,327 g |
| Levulose | 0,800 g |
| Acide citrique | 0,045 g |
| Citrate potassium | 0,0075 g |
| Hydroxyde de magnésie | 0,0050 g |
| Arôme orange | 0,0025 g |

**13.** Utilisation de la L-lysine pour potentialiser les effets biologiques de la L-carnitine dans la préparation d'un médicament destiné au traitement des pertubations musculaires liées à une hyperexcitabilité ou à une hypocontractilité.

**Patentansprüche**

**Patentansprüche für folgende Vertragsstaaten : DE, GB, FR, IT, NL, SE, CH, LI, BE, AT, LU**

**1.** Pharmazeutische und/oder diäthetische Zusammensetzungen, dadurch gekennzeichnet, daß sie als einzigen Wirkstoff L-Carnitin in Kombination mit L-Lysin oder einem Salz hiervon mit einer organischen oder anorganischen Säure in einer Menge enthalten, die eine Verstärkung der biologischen Wirkungen von L-Carnitin ergibt.

**2.** Zusammensetzungen nach Anspruch 1, dadurch gekennzeichnet, daß die angegebene Kombination durch Vermischen einer wäßrigen Lösung von L-Carnitin und von L-Lysin oder einem Salz hiervon mit einer organischen oder anorganischen Säure gebildet wird.

**3.** Zusammensetzungen nach Anspruch 1, dadurch gekennzeichnet, daß die angegebene Kombination durch ein Salz von L-Carnitin und von L-Lysin, beispielsweise durch das Hydrochlorid des L-Carnitinats von L-Lysin, gebildet wird.

**4.** Zusammensetzungen nach einem der Ansprüche 1 bis 3, dadurch gekennzeichnet, daß die Mengenverhältnisse von L-Carnitin und L-Lysin zwischen 1 : 0,5 und 1 : 20 Mol liegen.

**5.** Zusammensetzungen nach einem der Ansprüche 1 bis 4, dadurch gekennzeichnet, daß das Mengenverhältnis von L-Carnitin und L-Lysin äquimolar ist.

**6.** Zusammensetzungen nach einem der Ansprüche 1 bis 5, dadurch gekennzeichnet, daß sie in einer oral oder durch Injektion verabreichbaren Form vorliegen, die eine Tagesdosis von 1 bis 6 g einer äquimolaren Kombination von L-Carnitin und L-Lysin ergibt.

7. Zusammensetzungen nach einem der Ansprüche 1 bis 6, dadurch gekennzeichnet, daß sie in einer durch Injektion verabreichbaren Form mit folgender Zusammensetzung vorliegen:

| | |
|---|---|
| L-Carnitinbase | 0,500 g |
| L-Lysinhydrochlorid | 0,565 g |
| HCl q.s.p. | pH 4,5 |
| Wasser p.p.i. q.s.p. | 5 oder 10 ml |

Zur Anwendung als gelöster Stoff in einer Perfusion.

8. Zusammensetzungen nach einem der Ansprüche 1 bis 6, dadurch gekennzeichnet, daß sie als oral verabreichbare Formen mit folgender Zusammensetzung vorliegen:

| | |
|---|---|
| L-Carnitinbase | 0,500 g |
| L-Lysinhydrochlorid | 0,566 g |
| HCl q.s.p. | pH 4,5 |
| Natriumsaccharinat | 0,003 g |
| Orangenaroma | 0,020 g |
| gereinigtes Wasser q.s.p. | 5 ml |

9. Zusammensetzungen nach einem der Ansprüche 1 bis 6, dadurch gekennzeichnet, daß sie in Form einer Tropflösung mit folgender Zusammensetzung vorliegen:

| | |
|---|---|
| L-Carnitinbase | 15 g |
| L-Lysinhydrochlorid | 16,98 g |
| HCl q.s.p. | pH 4,5 |
| Natriumbenzoat | 0,10 g |
| gereinigtes Wasser q.s.p. | 100 ml |

als 90 ml fassendes Fläschchen mit einer Maßeinteilung von 2 ml (640 mg).

10. Zusammensetzungen nach einem der Ansprüche 1 bis 6, dadurch gekennzeichnet, daß sie in Form eines Säckchens mit folgender Zusammensetzung vorliegen:

| | |
|---|---|
| L-Carnitinbase | 0,5 g |
| L-Lysinhydrochlorid | 0,565 g |
| Zucker (Saccharose) | 3,920 g |
| Zitronenessenz | 0,015 g |

11. Zusammensetzungen nach einem der Ansprüche 1 bis 6, dadurch gekennzeichnet, daß sie in Form eines oral verabreichbaren Lyophilisats mit folgender Zusammensetzung vorliegen:

| | |
|---|---|
| L-Carnitinbase | 0,250 g |
| L-Lysinhydrochlorid | 0,283 g |
| Dextran 70 | 0,005 g |
| Xanthangummi | 0,005 g |
| Orangenaroma | 0,020 g |
| Zitronensäure | 0,005 g |
| Natriumsaccharinat | 0,003 g |
| Mannit | 0,429 g |
| Wasser | 0,500 g |

12. Zusammensetzungen nach einem der Ansprüche 1 bis 6, dadurch gekennzeichnet, daß sie in Form einer Kautablette mit folgender Zusammensetzung vorliegen:

| | |
|---|---|
| L-Carnitinbase | 0,250 g |
| L-Lysinhydrochlorid | 0,283 g |
| Maltodextrin | 0,500 g |
| Saccharose | 0,327 g |
| Lävulose | 0,800 g |
| Zitronensäure | 0,045 g |
| Kaliumcitrat | 0,0075 g |
| Magnesiumhydroxid | 0,0050 g |
| Orangenaroma | 0,0025 g |

13. Verfahren zur Herstellung einer pharmazeutischen und/ oder diäthetischen Zusammensetzung nach einem der Ansprüche 1 bis 12, dadurch gekennzeichnet, daß man L-Carnitin mit L-Lysin oder einem seiner Salze mit einer organischen oder einer anorganischen Säure in einer Menge, die eine Verstärkung der biologischen Wirkungen von L-Carnitin ergibt, vereinigt und diese Kombination mit pharmazeutisch und-/oder diäthetisch annehmbaren Hilfsstoffen vermischt.

14. Verwendung von L-Lysin zur Verstärkung der biologischen Wirkungen von L-Carnitin bei der Herstellung eines für die Behandlung von Muskelstörungen infolge einer Hypoexzitabilität oder einer Hypokontraktilität geeigneten Arzneimittels.

**Patentansprüche für folgende Vertragsstaaten : ES, GR**

1. Verfahren zur Herstellung einer pharmazeutischen und/ oder diäthetischen Zusammensetzung , dadurch gekennzeichnet, daß man L-Carnitin mit L-Lysin oder einem seiner Salze mit einer organischen oder einer anorganischen Säure in einer Menge, die eine Verstärkung der biologischen Wirkungen von L-Carnitin ergibt, vereinigt und diese Kombination mit pharmazeutisch und/oder diäthetisch annehmbaren Hilfsstoffen vermischt.

2. Verfahren nach Anspruch 1, dadurch gekennzeichnet, daß man L-Carnitin und L-Lysin oder eines seiner Salze mit einer organischen oder einer anorganischen Säure in wäßriger Lösung vermischt.

3. Verfahren nach Anspruch 1, dadurch gekennzeichnet, daß man ein Salz von L-Carnitin und von L-Lysin, beispielsweise das Hydrochlorid des L-Carnitinats von L-Lysin, bildet.

4. Verfahren nach einem der Ansprüche 1 bis 3, dadurch gekennzeichnet, daß man L-Carnitin und L-Lysin in Mengenverhältnissen kombiniert, die zwischen 1 : 0,5 und 1 : 20 Mol liegen.

5. Verfahren nach einem der Ansprüche 1 bis 4, dadurch gekennzeichnet, daß man L-Carnitin und L-Lysin in äquimolaren Mengenverhältnissen kombiniert.

6. Verfahren nach einem der Ansprüche 1 bis 5 zur Herstellung einer oral oder durch Injektion verabreichbaren Form, die eine Tagesdosis von 1 bis 6 g einer äquimolaren Kombination von L-Carnitin und L-Lysin ergibt.

7. Verfahren nach einem der Ansprüche 1 bis 6 zur Herstellung einer durch Injektion verabreichbaren Form folgender Zusammensetzung:

| | |
|---|---|
| L-Carnitinbase | 0,500 g |
| L-Lysinhydrochlorid | 0,565 g |
| HCl q.s.p. | pH 4,5 |
| Wasser p.p.i. q.s.p. | 5 oder 10 ml |

zur Anwendung als gelöster Stoff in einer Perfusion.

8. Verfahren nach einem der Ansprüche 1 bis 6 zur Herstellung von oral verabreichbaren Formen folgender Zusammensetzung:

| | |
|---|---|
| L-Carnitinbase | 0,500 g |
| L-Lysinhydrochlorid | 0,566 g |
| HCl q.s.p. | pH 4,5 |
| Natriumsaccharinat | 0,003 g |
| Orangenaroma | 0,020 g |
| gereinigtes Wasser q.s.p. | 5 ml |

9. Verfahren nach einem der Ansprüche 1 bis 6 zur Herstellung einer Zusammensetzung in Form einer Tropflösung mit folgender Zusammensetzung:

| | |
|---|---|
| L-Carnitinbase | 15 g |
| L-Lysinhydrochlorid | 16,98 g |
| HCl q.s.p. | pH 4,5 |
| Natriumbenzoat | 0,10 g |
| gereinigtes Wasser q.s.p. | 100 ml |

als 90 ml fassendes Fläschchen mit einer Maßeinteilung von 2 ml (640 mg).

10. Verfahren nach einem der Ansprüche 1 bis 9 zur Herstellung von Säckchen mit folgender Zusammensetzung:

| | |
|---|---|
| L-Carnitinbase | 0,5 g |
| L-Lysinhydrochlorid | 0,565 g |
| Zucker (Saccharose) | 3,920 g |
| Zitronenessenz | 0,015 g |

11. Verfahren nach einem der Ansprüche 1 bis 6 zur Herstellung eines oral verabreichbaren Lyophilisats mit folgender Zusammensetzung:

| | |
|---|---|
| L-Carnitinbase | 0,250 g |
| L-Lysinhydrochlorid | 0,283 g |
| Dextran 70 | 0,005 g |
| Xanthangummi | 0,005 g |
| Orangenaroma | 0,020 g |
| Zitronensäure | 0,005 g |
| Natriumsaccharinat | 0,003 g |
| Mannit | 0,429 g |
| Wasser | 0,500 g |

12. Verfahren nach einem der Ansprüche 1 bis 6 zur Herstellung einer Kautablette mit folgender Zusammensetzung:

| | |
|---|---|
| L-Carnitinbase | 0,250 g |
| L-Lysinhydrochlorid | 0,283 g |
| Maltodextrin | 0,500 g |
| Saccharose | 0,327 g |
| Lävulose | 0,800 g |
| Zitronensäure | 0,045 g |
| Kaliumcitrat | 0,0075 g |
| Magnesiumhydroxid | 0,0050 g |
| Orangenaroma | 0,0025 g |

13. Verwendung von L-Lysin zur Verstärkung der biologischen Wirkungen von L-Carnitin bei der Herstellung eines für die Behandlung von Muskelstörungen infolge einer Hypoexzitabilität oder einer Hypokontraktilität geeigneten Arzneimittels.

**Claims**

**Claims for the following Contrating States : DE, GB, FR, IT, NL, SE, CH, LI, BE, AT, LU**

1. Pharmaceutical and/or dietetic compositions, characterized in that they contain as sole active ingredient, L-carnitine, in combination with L-lysine or one of its organic or inorganic acid salts, in a quantity producing a potentiating effect of the biological effects of the L-carnitine.

2. Compositions according to claim 1, characterized in that the aforementioned combination is formed by the mixing in aqueous solution of L-carnitine and L-lysine or one of its organic or inorganic acid salts.

3. Compositions according to claim 1, characterized in that the aforementioned combination is formed by a salt of L-carnitine and L-lysine, for example the hydrochloride of the L-carnitine of L-lysine.

4. Compositions according to one of claims 1 to 3, characterized in that the relative proportions of L-carnitine and L-lysine are included, respectively, between 1:0.5 and 1:20 in moles.

5. Compositions according to any one of claims 1 to 4, characterized in that the relative proportion of L-carnitine and L-lysine is equimolar.

6. Compositions according to any one of claims 1 to 5, characterized in that they are available in a form compatible with oral or injectable administration at a daily dose of 1 to 6 g of the equimolar combination of L-carnitine and L-lysine.

7. Compositions according to any one of claims 1 to 6, characterized in that they are in an injectable form and in that they are made up of:

| | |
|---|---|
| L-carnitine base | 0.500 g |
| L-lysine hydrochloride | 0.565 g |
| HCl q.s.f. | pH 4.5 |
| Water p.f.i. q.s.f. | 5 or 10.- ml |

For use with a perfusion solution.

8. Compositions according to any one of claims 1 to 6, characterized in that they are in oral forms and in that they are made up of:

| | |
|---|---|
| L-carnitine base | 0.500 g |
| L-lysine hydrochloride | 0.566 g |
| HCl q.s.f | pH 4.5 |
| Saccharin Na | 0.003 g |
| Orange flavouring | 0.020 g |
| Purified water q.s.f. | 5.- ml |

9. Compositions according to any one of claims 1 to 6, characterized in that they are in the form of drops and in that they are made up of:

| | |
|---|---|
| L-carnitine base | 15.- g |
| L-lysine hydrochloride | 16.98 g |
| HCL q.s.f. | pH 4.5 |
| Sodium benzoate | 0.10 g |
| Purified water q.s.f. | 100.- ml |

Bottle of 90 ml with 2 ml (640 mg) measures.

10. Compositions according to any one of claims 1 to 6, characterized in that they are in the form of a sachet and in that they are made up of:

| | |
|---|---|
| L-carnitine base | 0.5 g |
| L-lysine hydrochloride | 0.565 g |
| Sugar (sucrose) | 3.920 g |
| Lemon oil | 0.015 g |

11. Compositions according to any one of claims 1 to 6, characterized in that they are in the form of an oral lyophilisate and in that they are made up of:

| | |
|---|---|
| L-carnitine base | 0.250 g |
| L-lysine hydrochloride | 0.283 g |
| Dextran 70 | 0.005 g |
| XANTHAN gum | 0.005 g |
| Orange flavouring | 0.020 g |
| Citric acid | 0.005 g |
| Saccharin Na | 0.003 g |
| Mannitol | 0.429 g |
| Water | 0.500 g |

12. Compositions according to any one of claims 1 to 6, characterized in that they are in the form of a chewable lozenge and in that they are made up of:

| | |
|---|---|
| L-carnitine base | 0.250 g |
| L-lysine hydrochloride | 0.283 g |
| Maltodextrin | 0.500 g |
| Sucrose | 0.327 g |
| Levulose | 0.800 g |
| Citric acid | 0.045 g |
| Potassium citrate | 0.0075 g |
| Magnesium hydroxide | 0.0050 g |
| Orange flavouring | 0.0025 g |

13. Process for the preparation of a pharmaceutical and/or dietetic composition according to one of claims 1 to 12, characterized in that L-carnitine is combined with L-lysine or one of its organic or inorganic acid salts, in a quantity producing a potentiating effect of the biological effects of L-carnitine and in that this combination is mixed with pharmaceutically and/or dietetically acceptable excipients.

14. Use of L-lysine to potentiate the biological effects of L-carnitine in the preparation of a medicine intended for the treatment of muscular disturbances associated with hypoexcitability or hypocontractility.

**Claims for the following Contrating States : ES, GR**

1. Process for the preparation of a pharmaceutical and/or dietetic composition, characterized in that it consists in combining L-carnitine with L-lysine or one of its organic or inorganic acid salts, in a quantity producing a potentiating effect of the biological effects of L-carnitine and in mixing this combination with pharmaceutically and/or dietetically acceptable excipient.

2. Process according to claim 1, characterized in that it comprises the mixture in aqueous solution of L-carnitine and L-lysine or one of its organic or inorganic acid salts.

3. Process according to claim 1, characterized in that it comprises the formation of a salt of L-carnitine and L-lysine, for example the hydrochloride of the L-carnitine salt of L-lysine.

4. Process according to one of claims 1 to 3, characterized in that L-carnitine and L-lysine are combined in relative proportions comprised between 1:0.5 and 1:20 expressed in moles.

5. Process according to one of claims 1 to 4, characterized in that L-carnitine and L-lysine are combined in equimolar proportions.

6. Process according to one of claims 1 to 5, for the preparation of a form compatible with oral or injectable administration at a daily dose of 1 to 6 g of the equimolar combination of L-carnitine and L-lysine.

7. Process according to one of claims 1 to 6, for the preparation of an injectable form comprising:

| | |
|---|---|
| L-carnitine base | 0.500 g |
| L-lysine hydrochloride | 0.565 g |
| HCl q.s.f. | pH 4.5 |
| Water p.f.i. q.s.f. | 5 or 10.- ml |

For use with a perfusion solution.

8. Process according to one of claims 1 to 6, for the preparation of oral forms, comprising:

| | |
|---|---|
| L-carnitine base | 0.500 g |
| L-lysine hydrochloride | 0.566 g |
| HCL q.s.f. | pH 4.5 |
| Saccharin Na | 0.003 g |
| Orange flavouring | 0.020 g |
| Purified water q.s.f. | 5.- ml |

9. Process according to one of claims 1 to 6, for the preparation in the form of drops comprising:

| | |
|---|---|
| L-carnitine base | 15.- g |
| L-lysine hydrochloride | 16.98 g |
| HCL q.s.f. | pH 4.5 |
| Sodium benzoate | 0.10 g |
| Purified water q.s.f. | 100.- ml |

Bottle of 90 ml with 2 ml (640 mg) measures.

10. Process according to any one of claims 1 to 6, for the preparation of sachets comprising:

| | |
|---|---|
| L-carnitine base | 0.5 g |
| L-lysine hydrochloride | 0.565 g |
| Sugar (sucrose) | 3.920 g |
| Lemon oil | 0.015 g |

11. Process according to any one of claims 1 to 6, for the preparation of an oral lyophilisate comprising

| | |
|---|---|
| L-carnitine base | 0.250 g |

| | |
|---|---|
| L-lysine hydrochloride | 0.283 g |
| Dextran 70 | 0.005 g |
| XANTHAN gum | 0.005 g |
| Orange flavouring | 0.020 g |
| Citric acid | 0.005 g |
| Saccharin Na | 0.003 g |
| Mannitol | 0.429 g |
| Water | 0.500 g |

12. Process according to any one of claims 1 to 6, for the preparation of a chewable lozenge comprising:

| | |
|---|---|
| L-carnitine base | 0.250 g |
| L-lysine hydrochloride | 0.283 g |
| Maltodextrin | 0.500 g |
| Sucrose | 0.327 g |
| Levulose | 0.800 g |
| Citric acid | 0.045 g |
| Potassium citrate | 0.0075 g |
| Magnesium hydroxide | 0.0050 g |
| Orange flavouring | 0.0025 g |

13. Use of L-lysine to potentiate the biological effects of L-carnitine in the preparation of a medicine intended for the treatment of muscular disturbances associated with hypoexcitability or hypocontractility.